# EUROPÄISCHE PATENTSCHRIFT

(11) **EP 1 350 533 B1**
(45) Veröffentlichungstag und Bekanntmachung des Hinweises auf die Patenterteilung: **24.11.2004**
(21) Anmeldenummer: 03090078.1
(22) Anmeldetag: 21.03.2003
(51) Int. Cl.: A61M 25/00

(54) **Oberflächenstruktur für eine Kathetergleitfläche**
Surface structure for a catheter sliding surface
Structure d'une surface pour la surface de glissement d'un cathéter

(30) Priorität: 21.03.2002 DE 10213368
(43) Veröffentlichungstag der Anmeldung: 08.10.2003
(73) Patentinhaber: BIOTRONIK Mess- und Therapiegeräte GmbH & Co Ingenieurbüro Berlin, 12359 Berlin (DE)
(72) Erfinder: Bertsch, Torben, 90403 Nürnberg (DE)
(74) Vertreter: Eisenführ, Speiser & Partner

(56) Entgegenhaltungen:
- EP-A- 0 384 476
- EP-A- 0 795 339
- DE-A- 3 045 237
- DE-A- 3 441 586
- US-A- 5 423 774

## Beschreibung

Die Erfindung betrifft eine Oberflächenstruktur für eine Reib- oder Gleitoberfläche eines Katheters oder Führungsdrahtes.

Katheter oder Führungsdrähte sind grundsätzlich bekannte medizinische Geräte, die in verschiedener Form zum Einführen in Körperhohlräume, wie beispielsweise Blutgefäße, ausgebildet sind. Derartige Katheter sind beispielsweise Katheter für die perkutane transluminale Coronar-Angioplastie (PTCA) oder auch Elektrodenleitungen für die Elektrostimulation, also beispielsweise für die intracardiale Defibrillation. Insbesondere Katheter zum Einführen in Blutgefäße weisen häufig eine große Länge auf und müssen den Verlauf der Blutgefäße bezüglich aller Abzweigungen und Windungen folgen, also flexibel sein. Ein solcher Katheter weist im implantierten Zustand über seine Länge eine Vielzahl von Biegungen auf.

Die hier interessierenden Katheter sind solche, die ein Lumen, also einen sich über die Länge des Katheters erstreckenden Hohlraum aufweisen, in den beispielsweise ein Führungsdraht, ein Mandrin oder ein Stilette oder eine andere Art von "Seele" längs- oder rotationsbeweglich einführbar ist. Insbesondere bei Kathetern mit vielen Biegungen ergibt sich das Problem, dass zwischen Reib- oder Gleitflächen des Katheters oder der Seele, also der Innenwand des Lumens und beispielsweise der Außenoberfläche der Seele, unerwünschte Reibungsverluste auftreten. Diese Reibungsverluste können beispielsweise die Präzision der Bewegung des Stilettes oder Führungsdrahtes im Katheter beeinträchtigen. Ein gleichartiges Problem ergibt sich bei relativ zueinander beweglichen Oberflächen der zur lateralen Auslenkung, beispielsweise eines Führungsdrahtes vorgesehenen Steuerdrähte im Inneren des Führungsdrahtes.

Der Erfindung liegt die Aufgabe zugrunde, präzisere Katheter der vorgenannten Art zu schaffen.

Diese Aufgabe wird bei einem Katheter oder Führungsdraht der eingangs genannten Art durch eine Oberflächenstruktur für eine Gleit- oder Reiboberfläche dieses Katheters oder Führungsdrahtes gelöst, welche sich durch eine Vielzahl von auf der Oberfläche verteilten Vertiefungen zur Aufnahme eines zur Reibungsminderung beitragenden Fluids sowie durch wenigstens eine Gleitfläche zwischen den Vertiefungen auszeichnet. Diese Gleitfläche begrenzt die Vertiefungen sowohl in Umfangsrichtung des Katheters oder Führungsdrahtes als auch in Längsrichtung. Die Vertiefungen sind somit nicht zusammenhängende kraterähnliche Vertiefungen, die in eine zusammenhängende Gleitfläche eingelassen sind.

In einer bevorzugten Ausführungsvariante ist das Verhältnis von größter zu kleinster Ausdehnung einer jeweiligen Vertiefung auf der Katheteroberfläche kleiner als 2. Unter Ausdehnung einer Vertiefung auf der Katheteroberfläche wird hierbei der Durchmesser der Vertiefung in Katheteroberflächenrichtung verstanden. Bei beispielsweise kreisrunden Vertiefungen gibt es nur einen Durchmesser, so dass das Verhältnis von größter zu kleinster Ausdehnung 1 ist. Ovale oder ellipsenförmige Vertiefungen können ein anderes Durchmesserverhältnis haben, dass jedoch nicht größer als 2 sein soll.

Der Umriss der Vertiefungen auf der Katheteroberfläche ist vorzugsweise rundlich, also beispielsweise kreisrund, oval oder ellipsenförmig.Als zur Reibungsminderung beitragendes Fluid ist beispielsweise eine Kochsalzlösung geeignet, da diese physiologisch verträglich eingestellt werden kann. Andere, vorzugsweise physiologisch verträgliche Fluide oder beispielsweise eine Kochsalzlösung und eventuell ein Kontrastmittel enthaltende Fluidmischungen kommen ebenfalls in Frage.

Die erfindungsgemäße Oberflächenstruktur ist im weitesten Sinne überall dort vorteilhaft einsetzbar, wo bei flexiblen medizinischen Geräten innerhalb der Kontaktzonen Reib- oder Gleitbewegungen zwischen oder auf Oberflächen an Bestandteilen des medizinischen Gerätes auftreten.

Vorzugsweise sind die Vertiefungen über die Reib- oder Gleitoberfläche gleichmäßig verteilt, wobei die Vertiefungen sich besonders bevorzugt in ihrer Größe voneinander unterscheiden. Diese Verteilung der unterschiedlich großen Vertiefungen ist vorzugsweise unregelmäßig. Es wird somit eine zwar gleichmäßige, aber nicht strengen Regeln folgende, systematische, sondern beispielsweise quasi-zufällige Verteilung der verschieden großen Vertiefungen über die Reib- oder Gleitoberfläche bevorzugt.

Je nach Anwendung kann aber auch eine regelmäßige oder systematische Verteilung der Vertiefungen vorteilhaft sein.

Die Übergänge zwischen den Gleitflächen zwischen den Vertiefungen und den Vertiefungen selbst sind vorzugsweise abgerundet.

In einer bevorzugten Ausführungsvariante ist die zuvor beschriebene Oberflächenstruktur auf der Innenseite eines Lumens eines Katheters vorgesehen.

Alternativ ist die beschriebene Oberflächenstruktur für die als Gleitoberfläche dienende Außenoberfläche eines Führungsdrahtes vorgesehen.

Ebenso kann die Oberflächenstruktur für eine Gleitoberfläche eines Stilettes oder Mandrins vorgesehen sein. Im letztgenannten Fall muss es sich nicht nur um die Gleitoberfläche zwischen beispielsweise Stilette oder Mandrin und der Innenwand eines Lumens eines Katheters handeln, vielmehr kann die Gleitoberfläche mit der entsprechenden Oberflächenstruktur auch eine der Gleitflächen zwischen den zur Auslenkung oder Steuerung dienenden Bestandteilen eines Stilettes, Mandrins oder Führungsdrahtes sein.

Die Erfindung soll nun anhand eines Ausführungsbeispiels mit Hilfe der Figuren näher erläutert werden.

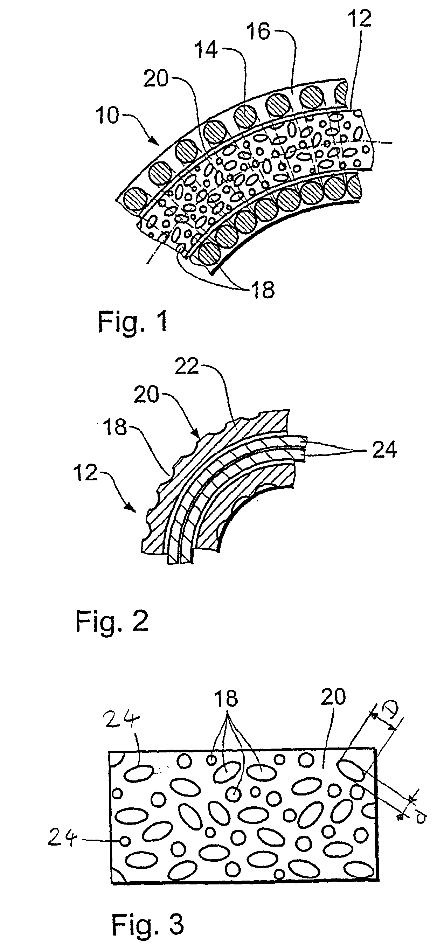

Von den Figuren zeigt Figur 1 einen Abschnitt eines Katheters mit einem Lumen und darin befindlichem Führungsdraht in teilweiser geschnittener Darstellung.
- Figur 2: einen Längsschnitt durch einen Abschnitt eines Führungsdrahtes und
- Figur 3: eine beispielhafte Darstellung der Abwicklung einer geeigneten Oberflächenstruktur.

Figur 1 zeigt einen Abschnitt eines Katheters 10 mit einem darin befindlichen Abschnitt eines Führungsdrahtes 12. Der Katheterabschnitt 10 ist dabei geschnitten dargestellt, während der Führungsdraht 12 nicht geschnitten dargestellt ist.

Der Katheterabschnitt 10 wird von einer Metallhelix 14 gebildet, die in elastisches Wandmaterial 16, beispielsweise Silicon enthaltend, eingebettet ist. Das Wandmaterial 16 mit darin eingebetteter Metallhelix 14 schließen einen Hohlraum ein, der ein Lumen für den Führungsdraht 12 bildet.

Der Führungsdraht 12 besitzt auf seiner Außenseite eine Oberflächenstruktur, die von gleichmäßig, aber nicht regelmäßig verteilten Vertiefungen 18 unterschiedlicher Größe gebildet ist. Die Abmessungen der Vertiefungen 18 sind dabei so gewählt, das jede Vertiefung sich in Umfangsrichtung des Führungsdrahtes 12 nur über einen Bruchteil dieses Umfangs erstreckt, beispielsweise über weniger als 1/10 des Umfangs. Der Bruchteil hängt maßgeblich vom Gesamtumfang der Gleitoberfläche ab und ist mit zunehmendem Umfang oder Durchmesser tendenziell kleiner zu wählen.

Die Vertiefungen 18 sind vorzugsweise von rundlicher Form, beispielsweise kreis- oder ellipsenförmig.

Zwischen dem Katheterabschnitt 10 und dem Führungsdraht 12 ist mit Fluid gefüllter Zwischenraum vorgesehen. Dieses Fluid ist über die Vertiefungen 18 verteilt, so dass die Vertiefungen 18 kleine Vorräte des Fluids enthalten. Das Fluid setzt die Reibung zwischen den Gleitflächen 20 des Führungsdrahtes 12 und der Innenwand des Lumens des Kathters 10 herab. Die Gleitflächen haben daher eine den Fluidfilm tragende Funktion und sind entsprechend zu bemessen.

Ein geeignetes Fliud ist physiologisch verträgliche Kochsalzlösung.

Der Übergang zwischen den Vertiefungen 18 und den zwischen den Vertiefungen befindlichen Gleitflächen 20 ist abgerundet.

Figur 2 zeigt einen Führungsdraht 12 ähnlich dem aus Figur 1, diesmal jedoch in geschnittener Darstellung. Zu erkennen ist eine Hülle 22 des Führungsdrahtes 12, die auf ihrer Außenoberfläche Vertiefungen 18 trägt und dazwischen angeordnete Gleitflächen 20.

Die Hülle 22 schließt ebenfalls einen längsgestreckten Hohlraum ein, die beispielsweise von flachen Metallbändern gebildete Steuermittel 24 zur abgebildeten lateralen Auslenkung des Führungsdrahtes 12 beherbergt. Die Steuermittel 24 sind ausgebildet, um durch axiales relatives Verschieben zueinander eine seitliche Auslenkung des Führungsdrahtes 12 zu bewirken. Mithin ergibt sich zwischen den beiden Steuermitteln 24 eine Gleitfläche. Eine der diese Gleitfläche bildenden Oberflächen der Steuermittel 24 kann ebenfalls mit einer Oberflächenstruktur, wie zuvor beschrieben, versehen sein.

Figur 3 zeigt eine geeignete Oberflächenstruktur für Gleit- oder Reibflächen flexibler medizinischer Geräte in vergrößerter Darstellung. Insbesondere ist die zwar unregelmäßige, aber gleichmäßige Verteilung der unterschiedlich großen Vertiefungen dargestellt. Um eine gleichmäßige aber unregelmäßige Verteilung vorzusehen, können die unterschiedlich großen Vertiefungen 18 quasi-zufällig über die Oberfläche verteilt sein.

Figur 3 ist zu entnehmen, dass die Vertiefungen 18 sämtlich kreisförmige oder ellipsenförmige bzw. ovale Umrisse 24 haben. Außerdem ist für eine Vertiefung deren größte Ausdehnung D auf der Katheteroberfläche und deren kleinste Ausdehnung d auf der Katheteroberfläche eingezeichnet. Das Verhältnis von D/d ist vorzugsweise nicht sehr viel größer als 2 und besonders bevorzugt zwischen 1 und 2.

Der in Figur 1 abgebildete Katheter 10 ist vorzugsweise entweder eine Elektrodenleitung für einen Herzschrittmacher oder Defibrillator oder aber auch ein Katheter für das intraluminale Platzieren von Prothesen wie Stents oder Ähnlichem.

## Patentansprüche

1. Oberflächenstruktur für eine Reib- oder Gleitoberfläche eines Katheters (10) oder Führungsdrahtes (12) mit einer Längs- und einer Umfangsrichtung , **gekennzeichnet durch** eine Vielzahl auf der Oberfläche verteilter Vertiefungen (18) zur Aufnahme eines zur Reibungsminderung beitragenden Fluids sowie **durch** wenigstens eine zusammenhängende Gleitfläche (20) zwischen den Vertiefungen (18),wobei die Vertiefungen bezüglich ihrer Abmessungen auf der Katheteroberfläche sowohl in Längs- als auch in Umfangsrichtung **durch** die Gleitfläche (20) begrenzt sind.

2. Oberflächenstruktur nach Anspruch 1, **dadurch gekennzeichnet, dass** das Verhältnis von größter Ausdehnung einer jeweiligen Vertiefung auf der Katheteroberfläche zur kleinsten Ausdehnung dieser Vertiefung auf der Katheteroberfläche kleiner ist als 2.

3. Oberflächenstruktur nach Anspruch 1, **dadurch gekennzeichnet, dass** die Vertiefungen (18) über die Reib- oder Gleitoberfläche gleichmäßig verteilt und verschieden groß sind.

4. Oberflächenstruktur nach Anspruch 3 **dadurch gekennzeichnet, dass** die Verteilung der unterschiedlich großen Vertiefungen (18) unregelmäßig ist.

5. Oberflächenstruktur nach einem der Ansprüche 1 bis 4, **gekennzeichnet durch** abgerundete Übergänge zwischen den Gleitflächen (20) und den Vertiefungen (18).

6. Oberflächenstruktur nach einem der Ansprüche 1 bis5, **dadurch gekennzeichnet, dass** die Abmessungen der Vertiefungen (18) in Umfangsrichtung der Reib- oder Gleitoberfläche kleiner sind, als 1/10 des Umfangs.

7. Oberflächenstruktur nach einem der Ansprüche 1 bis 6, **dadurch gekennzeichnet, dass** die Vertiefungen (18) eine runde Form besitzen und vorzugsweise kreis- oder elipsenförmig ausgebildet sind.

8. Katheter (10) mit einem Lumen, welches eine innenseitige Oberfläche aufweist, **dadurch gekennzeichnet, dass** die innenseitige Oberfläche gemäß einem der Ansprüche 1 bis 7 ausgebildet ist.

9. Katheter (10), vorzugsweise mit einem Lumen, welcher eine Außenoberfläche aufweist, **dadurch gekennzeichnet, dass** die Außenoberfläche gemäß einem der Ansprüche 1 bis 7 ausgebildet ist.

10. Führungsdraht (12) mit einer Außenoberfläche, die als Gleitoberfläche nach einem der Ansprüche 1 bis 8 ausgebildet ist.

11. Stilette oder Mandrin mit einer Außenoberfläche, die nach einem der Ansprüche 1 bis 8 ausgebildet ist.

## Claims

1. Surface structure for a frictional or sliding surface of a catheter (10) or guide wire (12) having a longitudinal and peripheral direction, **characterised by** a plurality of recesses (18) distributed on the surface for receiving a fluid contributing to friction reduction, as well as by at least one connected sliding surface (20) between the recesses (18), wherein the recesses, with regard to their dimensions are delimited by the sliding surface (20) on the catheter surface both in the longitudinal and peripheral direction.

2. Surface structure as claimed in Claim 1, **characterised in that** the ratio of the largest dimension of a respective recess on the catheter surface to the smallest dimension of this recess on the catheter surface is smaller than 2.

3. Surface structure as claimed in Claim 1, **characterised in that** the recesses (18) are evenly distributed over the frictional or sliding surface and are different in size.

4. Surface structure as claimed in Claim 3, **characterised in that** the recesses (18), which are of different sizes, are distributed in an irregular manner.

5. Surface structure as claimed in any one of Claims 1 to 4, **characterised by** rounded-off transitions between the sliding surfaces (20) and the recesses (18).

6. Surface structure as claimed in any one of claims 1 to 5, **characterised in that** the dimensions of the recesses (18) in the peripheral direction of the frictional or sliding surface are smaller than 1/10 of the perimeter.

7. Surface structure as claimed in any one of Claims 1 to 6, **characterised in that** the recesses (18) have a round shape and are preferably formed so as to be circular or elliptical.

8. Catheter (10) having a lumen which comprises an inner surface, **characterised in that** the inner surface is formed in accordance with any one of Claims 1 to 7.

9. Catheter (10), preferably having a lumen which comprises an outer surface, **characterised in that** the outer surface is formed in accordance with any one of Claims 1 to 7.

10. Guide wire (12) having an outer surface which is formed as a sliding surface as claimed in any one of Claims 1 to 8.

11. Stylet or mandrel having an outer surface which is formed in accordance with any one of claims 1 to 8.

## Revendications

1. Structure de surface pour une surface de friction ou de glissement d'un cathéter (10) ou d'un fil de guidage (12), avec une direction longitudinale et une direction périphérique, **caractérisée par** une multitude de renfoncements (18) répartis sur la surface et destinés à recevoir un fluide contribuant à réduire la friction, ainsi que par au moins une surface de glissement cohérente (20) entre les renfoncements (18), les renfoncements étant délimités quant à leurs dimensions sur la surface de cathéter aussi bien en direction longitudinale qu'en direction périphérique par la surface de glissement (20).

2. Structure de surface selon la revendication 1, **caractérisée en ce que** le rapport de l'extension maximale d'un renfoncement respectif sur la surface de cathéter et de l'extension minimale de ce renfoncement sur la surface de cathéter est inférieur à 2.

3. Structure de surface selon la revendication 1, **caractérisée en ce que** les renfoncements (18) sont répartis de façon régulière sur la surface de friction ou de glissement et présentent des tailles différentes.

4. Structure de surface selon la revendication 3, **caractérisée en ce que** la répartition des renfoncements (18) présentant des tailles différentes est irrégulière.

5. Structure de surface selon l'une des revendications 1 à 4, **caractérisée par** des transitions arrondies entre les surfaces de glissement (20) et les renfoncements (18).

6. Structure de surface selon l'une des revendications 1 à 5, **caractérisée en ce que** les dimensions des renfoncements (18) en direction périphérique de la surface de friction ou de glissement sont inférieures à un dixième de la circonférence.

7. Structure de surface selon l'une des revendications 1 à 6, **caractérisée en ce que** les renfoncements (18) présentent une forme ronde et sont réalisés de préférence sous forme circulaire ou elliptique.

8. Cathéter (10) avec un lumen qui présente une surface côté intérieur, **caractérisé en ce que** la surface côté intérieur est réalisée selon l'une des revendications 1 à 7.

9. Cathéter (10) de préférence avec un lumen qui présente une surface extérieure, **caractérisé en ce que** la surface extérieure est réalisée selon l'une des revendications 1 à 7.

10. Fil de guidage (12) avec une surface extérieure qui est réalisée sous forme de surface de glissement selon l'une des revendications 1 à 8.

11. Stylet ou mandrin avec une surface extérieure qui est réalisée selon l'une des revendications 1 à 8.
